# EUROPEAN PATENT APPLICATION

(11) **EP 1 125 910 A1**
(43) Date of publication of application: **22.08.2001**
(21) Application number: 00500024.5
(22) Date of filing: 18.02.2000
(51) Int. Cl.: C07C 45/86, C07C 47/04, C07C 47/058, A01N 35/02, A61L 2/18, A01K 1/01

(54) **New stabilized aqueous formaldehyde solution, process for the preparation thereof and uses**

(71) Applicant: Poblador Sanmiguel, Miguel Angel, 31512 Fontellas (Navarra) (ES)
(72) Inventor: Poblador Sanmiguel, Miguel Angel, 31512 Fontellas (Navarra) (ES)
(74) Representative: Ungria Lopez, Javier

(57) **Abstract**

The solution is comprised of:

| | |
|---|---|
| BASIC FORMALDEHYDE SOLUTION | 35% ± 5% (w/w) |
| GUM XANTHANE | 0.09% ± 0.05% (w/w) |
| ESSENCE | 1% ± 0.5% (w/w) |
| DYE | 0.3% ± 0,1% (w/w) |
| WATER | 63.7% ± 5% (w/w) |

The process essentially comprises the mixing of the different components with water at 45-55°C stirring until the perfect homogenization thereof is achieved.

Use in live-stock breeding, agriculture and other industrial sectors.

## Description

The present invention fits in the industry of formaldehyde and its uses.

Specifically, the present invention provides a new stabilized formaldehyde solution with important uses in industry in general and in particular in agriculture, live-stock breeding and medicine.

### PRIOR ART OF THE INVENTION

Formaldehyde with a semi-empirical formula H-CHO is an easily polymerizable gas, widely known and used in multiples uses, for more than 90 years.

Commercially it tends to be in a 37-50% aqueous solution form, containing stabilizers in order to inhibit the polymerization of the formaldehyde. A conventionally used stabilizer is methanol. These stabilized aqueous formaldehyde solutions are commonly known as formalins.

Formaldehyde has a boiling point of -19°C and a melting point of -118°C, its autoignition point being 430°C.

From a toxicological point of view it should be said that formaldehyde is an intermediary essential in animal and human metabolism, it being present in human blood in concentrations of around 2-3 ppm. Besides it has the advantage that it does not accumulate in the body because it is rapidly oxidized and biodegraded. For example, it can be said that in human metabolism it has an average life of 1.5 minutes, mainly decomposing into carbon dioxide and water.

Formaldehyde does not cause any allergic reaction or any type of harmful action for the lungs, since it metabolizes and degrades in the respiratory tract within a few seconds. Only prolonged exposure to formaldehyde solutions, or to gaseous formaldehyde, can produce a certain degree of irritation to the skin, eyes and mucous membranes. Aside from this, no harmful effects for health have been described for formaldehyde.

From the environmental point of view, it is known that formaldehyde is a gas present in the atmosphere in small concentrations (0.005-0.165 ppm) as a result of incomplete combustion, for example, gases from vehicles, industrial combustion and even cigarette smoke.

The formaldehyde present in the atmosphere is in continuous degradation, transforming into carbon dioxide and water, this degradation being influenced by sunlight and nitrogen oxides.

It is for this reason that formaldehyde is a product that does not accumulate in the atmosphere at levels in which it could prove to be toxic (the described tolerance level in air is 5 ppm).

Besides, used as a solution it is biodegradable, with a very short average life, after which it completely disappears from the medium. This advantage, together with those cited above, make formaldehyde a product very suitable for multiple agricultural, live-stock breeding, industrial, medical uses, etc., some of which are stated in the following paragraphs. Formaldehyde has a high germicide level for which reason it is used as a disinfectant in different sectors.

Formaldehyde has also been used in fluids for embalming, as a preservative, hardener, corrosion inhibitor, etc.

There are a multitude of patents related to formaldehyde and its wide diversity of uses. A brief explanation of some of them is made hereinafter due to their closer relationship to the object of the present invention.

Patent E87116781 refers to a process for cleaning and disinfecting endoscopes and to a method to carry out said process. For this purpose, the surfaces of the endoscope are first put in contact with a cleaning solution at 55-65°C, after which they are put in contact with a disinfecting medium at 55-65°C and finally they are rinsed. Said disinfecting medium contains an aldehyde selected from among formaldehyde and aliphatic dialdehydes with 2-8 carbon atoms and it has a pH of 6-8.

Patent E89108379, along the same lines as the preceding one, reveals a process for cleaning and disinfecting heat sensitive medical instruments, especially, endoscopes. A cleansing and disinfecting solution that comprises, among other components, a proteolytic enzyme, a complex, an aldehyde selected from among formaldehyde and aliphatic aldehydes with 2-8 carbon atoms, is used in said process.

Patent P9301542 refers to a disinfectant for devices for access to live tissues and to a process for disinfecting said devices. The disinfectant that is applied to the device is a substance of an organic chemical nature with one or two carbon atoms in its molecule, metabolizable by the live tissues, active in an organic medium, stable at room temperature and in daylight, not accumulable in the live tissues, harmless for the material constituting the devices to be treated, and destructive of bacteria, viruses, fungi and spores, for example, formaldehyde or a mixture of formaldehyde and ethanol. The patent is especially applicable to implanted venous access devices.

Patent E94250040 describes a concentrate of an aqueous disinfecting medium that comprises glutaric aldehyde and/or formaldehyde, one or more alcohols with a limited capacity of mixing with water and possibly with conventional auxiliary substances. In order to be used, the concentrate is diluted in water. It is established that the advantages of this concentrate basically lie on its reduced odor, its greater tolerability on the part of materials, its improved microbicidal activity and its greater stability with respect to storage and with respect to other already existing similar products. Said concentrate is especially used to disinfect medical and surgical instruments as well as different types of surfaces exposed to contamination by germs of a varied etiology.

In another scope of use, formaldehyde has been used as an unpleasant odor controlling agent. Hence, for example, patent P9201230 refers to an insole for footwear the constituent part of which includes a formulation based on boric acid, aluminum and potassium sulfate, formaldehyde and quaternary ammonium compounds. The insole thus formed is used to neutralize sweat and sweaty odors of the feet and to destroy the germs on the skin.

Formaldehyde is also used in the food industry sector. Hence, patent E93403112 describes food for live-stock resistant to contamination. The food is treated with formaldehyde, which as it has already been indicated, is resistant to contamination by pathogenic bacteria, by means of spraying the same with an aqueous solution that contains 10-50% by weight of formaldehyde.

Another use of formaldehyde is its capacity to degrade different types of fibers. Patent E93913772 refers to a process to degrade a polymer that comprises the reaction of the polymer with formaldehyde in a suitable medium. The descaling capacity of formaldehyde is also noteworthy, a capacity that is manifested, for example, in patent P8101652 wherein a sulfurated scale is eliminated by treating it with an aqueous composition constituted by an aqueous non-oxidizing acid, an aldehyde (for example, formaldehyde generated in situ) and an amine type acid corrosion inhibitor.

This brief review of the literature related to formaldehyde can serve as a sample in order to manifest the wide diversity of industrial sectors in which formaldehyde is useful. Likewise, it manifests that there is ample concern for this product and that there are many companies that manufacture under a patent formaldehyde solutions of different compositions.

Obviously, the basic problem is always the stability of said solutions, since formaldehyde spontaneously polymerizes into paraformaldehyde. Paraformaldehyde can be used on occasions instead of formaldehyde for the same purposes, but not on other occasions.

Due to all of the above, it proves to be logical and evident that formaldehyde suppliers continue doing research in order to offer the market stabler solutions with improved properties for the different uses thereof.

Along these lines the present invention provides a new especially stable aqueous formaldehyde solution with a wide capacity of use for very different uses in the field of medicine, agriculture, food, etc.

### DETAILED DESCRIPTION OF THE INVENTION

As indicated in its title, the present invention refers to a new stabilized aqueous formaldehyde solution, to the process for the preparation thereof and its uses.

The new aqueous formaldehyde solution of the present invention is characterized in that it comprises the following components in the following amounts

| | |
|---|---|
| BASIC FORMALDEHYDE SOLUTION | 35% ± 5% (w/w) |
| GUM XANTHANE | 0.09% ± 0.05% (w/w) |
| ESSENCE | 1% ± 0.5% (W/W) |
| DYE | 0.3% ± 0,1% (w/w) |
| WATER | 63.7% ± 5% (w/w) |

A 27% (w/w) commercial aqueous solution stabilized with 13% (w/w) methanol is used as the basic formaldehyde solution.

The CARIBE 1200/DS brand product made from a multitude of odorous products of a different nature tends to be used as the essence.

OPACIFIER 621, which is an 0.1% (w/w) aqueous sty-rene solution, depending on the manufacturer's specifications, is normally used as the dye.

The solution thus formed has the following physical and chemical properties:

| | |
|---|---|
| APPEARANCE | Viscous liquid |
| COLOR | Bluish white |
| SMELL | Spicy like formol |
| pH | 6.5-7.5 |
| FLASH POINT | Non-inflammable |
| BOILING POINT | 100°C±1°C |
| DENSITY | 1.02 ± 0.01 g/cc |
| SOLUBILITY | Water-soluble in all proportions |

The process for the preparation of the solution of the invention, comprises the following phases:

### PHASE 1

1) The necessary amount of water at a temperature of 45-55°C is placed in a hopper.
2) Gum xanthane is prepared. The following steps are followed for this purpose:
   2.1) The exact amount of gum xanthane is placed in a tank, preferably made out of plastic.
   2.2) A small amount of alcohol selected between isopropanol and ethanol or a mixture thereof in any proportion is added to it. The proportion of alcohol to be used is the minimum necessary to be able to work with gum xanthane and to make its dissolving in water possible, which would otherwise be very difficult. This proportion is in the range of 0.25-0-40 g of alcohol per gram of gum xanthane.
   2.3) This is stirred with a rod until a homogenous paste is obtained.
3) The predispersed gum xanthane is poured into the hopper, beginning to stir for 15-30 minutes, until the gum has totally dissolved.
4) Then the essence and dye are added.
5) The mixture is kept stirred for 15-30 minutes until the perfect homogenization thereof is obtained.

### PHASE 2

The indicated amount of formaldehyde in the form of a 27% aqueous solution is placed in a container.

### PHASE 3

The product resulting from phase 1 is added to phase 2 and this is stirred until a perfect mixture is obtained.

The solution thus obtained has a sophisticated and ecological formulation that makes it especially ideal to provide a rapid and effective solution for the problems of:
- Agricultural, live-stock breeding or industrial disinfection
- Treatment and elimination of odors
- Degradation and liquefaction of organic matter (purines, fatty acids, polymeric material, organic wastes, etc.)

### Use as a disinfectant

Present-day needs regarding hygiene material require disinfectants to prevent the growth of microorganisms that can produce substances that are extremely dangerous for human beings.

As a microbial agent, the formaldehyde solution of the present invention has a very broad action spectrum, also having the advantage, with respect to other disinfectants, as it is a product that is harmless to health and the environment as it has already been stated.

### Use as an odor preventive agent

The formaldehyde solution of the present invention is capable of eliminating a high percentage of gases resulting from the degradation of organic matter (for example, ammonia, sulfuric acid, methane, etc.) transforming them into harmless and/or biodegradable products.

As it also eliminates the microorganisms that produce these gases, its double action allows the practically total elimination of bad odors present in organic wastes.

### Use as a degradating agent of organic matter

The organic matter degradating capacity that the solution of the invention has, can be summarized in the following aspects:
- Degradation of the cellulose fibers present in purines, which helps to liquefy the waste, facilitating the conveyance thereof by pumping or by gravity.
- Chemical degradation of the fatty acids present in organic wastes, generating biodegradable compounds.
- Degradation of proteins and carbohydrates present in organic wastes.

Due to all of the above, the new solution of the present invention is especially suitable for use in live-stock breeding farms where it is necessary to treat and eliminate daily large amounts of purines, excrements, and animal wastes. With the solution of the invention, it is possible to liquefy excrements, degradate them chemically and to control the odor thereof.

### EMBODIMENTS OF THE INVENTION

The present invention is additionally illustrated by means of the following example, which by no means tries to be restrictive of its scope.

### EXAMPLE

A formaldehyde solution was prepared from 35 kg of formaldehyde (27% (w/w) commercial aqueous solution stabilized with 13% (w/w methanol), 90 g of gum xanthane, 1 kg. of Caribe 1200, 300 g of Opacifier 621 and 64 kg. of water.

For this purpose, the following phases were followed:

### Phase 1:

1) The water at a temperature of 50°C was placed in a hopper.
2) The gum xanthane was prepared by the following steps:
   2.1) The exact amount of gum xanthane was put in a plastic tank
   2.2) A small amount of mostanol alcohol (mostanol alcohol is a mixture of 63% ethyl alcohol and 37% isopropanol v/v) was added to it.
   2.3) It was stirred with a rod until a homogenous paste has been obtained.
3) The predispersed gum xanthane was poured into the hopper, beginning to stir for 25 minutes, until the gum has been totally dissolved.
4) Then the essence and dye were added.
5) The mixture is kept stirred for 15 minutes until the perfect homogenization thereof is achieved.

### Phase 2:

The indicated amount of formaldehyde in the form of a 27% aqueous solution is placed in a container.

### Phase 3:

The product resulting from phase 1 is added to phase 2 and this is stirred until a perfect mixture is obtained.

The formaldehyde solution thus obtained, that we will call "formil" hereinafter, was used to clean and disinfect the pits for collecting excrements of animals of a live-stock breeding farm, and the corresponding gratings thereof.

For this purpose, an initial treatment was carried out and then a maintenance treatment:
- Initial treatment: 7 liters of "formil" were used for every 200 liters of water in order to hose down a grating surface of 200m². The gratings were hosed down with this preparation once a week for three weeks.
- Maintenance treatment: 3.5 liters of "formil" were used for every 200 liters of water in order to hose down said grating surface of 200 m². The gratings were hosed down with this preparation once a month.

In this way degradation and liquefaction of the excrements accumulated in the pits were achieved, permitting the easy cleaning thereof. Besides, the odor is perfectly controlled, practically achieving the total elimination thereof.

## Claims

1. New stabilized aqueous formaldehyde solution, characterized in that it comprises the following components in the following amounts:
| | |
|---|---|
| BASIC FORMALDEHYDE SOLUTION | 35% ± 5% (w/w) |
| GUM XANTHANE | 0.09% ± 0.05% (w/w) |
| ESSENCE | 1% ± 0.5% (w/w) |
| DYE | 0.3% ± 0,1% (w/w) |
| WATER | 63.7% ± 5% (w/w) |

2. Solution, according to claim 1, characterized in that the basic formaldehyde solution is a 27% (w/w) commercial aqueous formaldehyde solution stabilized with 13% (w/w) of methanol.

3. Solution, according to claim 1, characterized in that the dye is a 0.1% (w/w) commercial aqueous sty-rene solution.

4. Solution, according to claim 1, characterized in that it has the following physical and chemical properties:
| | |
|---|---|
| APPEARANCE | Viscous liquid |
| COLOR | Bluish white |
| SMELL | Spicy like formol |
| pH | 6.5-7.5 |
| FLASH POINT | Non-inflammable |
| BOILING POINT | 100°C±1°C |
| DENSITY | 1.02 ± 0.01 g/cc |
| SOLUBILITY | Water-soluble in all proportions |

5. Process for the preparation of the aqueous formaldehyde solution of claim 1, characterized in that it comprises the following phases:
Phase 1:
a) place the water at a temperature of 45-55°C in a hopper;
b) predisperse the gum xanthane in an alcohol selected from among ethanol, isopropanol or a mixture thereof;
c) pour the gum xanthane predispersed in the alcohol in the hopper, stirring for 15-30 minutes, until the gum has totally dissolved;
d) then add the essence and the dye;
e) keep the mixture stirred for 15-30 minutes until the perfect homogenization thereof is achieved;
Phase 2: place the formaldehyde in said form of 27% (w/w) stabilized aqueous solution in a container;
Phase 3: add the product resulting from phase 1 to phase 2 and stir until a perfect mixture is achieved.

6. Use of the solution of claims 1 to 4, for the manufacture of disinfecting agents for agricultural, live-stock breeding or industrial disinfection.

7. Use of the solution of claims 1 to 4 for the manufacture of agents for the treatment and elimination of odors.

8. Use of the solution of claims 1 to 4, for the manufacture of agents for the degradation and liquefaction of organic matter.

9. Use of the solution of the claims 1 to 4, for the manufacture of agents for the treatment and elimination of excrements, purines and other animal wastes in the pits for collecting the same of live-stock breeding farms.
